# EUROPÄISCHE PATENTSCHRIFT

(11) **EP 2 269 662 B1**
(45) Veröffentlichungstag und Bekanntmachung des Hinweises auf die Patenterteilung: **26.02.2014**
(21) Anmeldenummer: 10006051.6
(22) Anmeldetag: 11.06.2010
(51) Int. Cl.: B23K 1/005, A61B 18/18, A61L 24/00, A61L 26/00, A61B 17/00, A61K 9/51, B23K 35/36, B29C 65/14, B29C 65/48

(54) **Lot zum stoffschlüssigen Fügen zweier Oberflächen**
Solder for strong connection of two surfaces
Soudure destinée à l'assemblage interposé de deux surfaces

(30) Priorität: 30.06.2009 DE 102009031261
(43) Veröffentlichungstag der Anmeldung: 05.01.2011
(73) Patentinhaber: Storz Endoskop Produktions GmbH, 78532 Tuttlingen (DE)
(72) Erfinder: Krattiger, Beat, 8222 Beringen (CH)
(74) Vertreter: Fugmann, Winfried

(56) Entgegenhaltungen:
- EP-A1- 0 160 752
- EP-A2- 0 251 257
- WO-A1-01/30410
- WO-A1-96/22797
- US-A- 3 742 181
- US-A1- 2007 260 325
- PARK J W ET AL: "Joint structure in high brightness light emitting diode (HB LED) packages", MATERIALS SCIENCE AND ENGINEERING A: STRUCTURAL MATERIALS:PROPERTIES, MICROSTRUCTURE & PROCESSING, LAUSANNE, CH, vol. 441, no. 1-2, 15 December 2006 (2006-12-15), pages 357-361, XP027953391, ISSN: 0921-5093 [retrieved on 2006-12-15]

## Beschreibung

Die vorliegende Erfindung bezieht sich auf ein Verfahren und ein Lot zum stoffschlüssigen Fügen zweier Oberflächen, insbesondere an oder in einem menschlichen oder tierischen Körper.

Bei endoskopischen und konventionellen Operationen müssen Oberflächen gefügt bzw. dauerhaft mechanisch verbunden werden. Beispielsweise werden Öffnungen verschlossen, Gefäße, Sehnen oder Nerven miteinander verbunden. Lange Zeit wurden dazu ausschließlich mittels Nadel und Faden Nähte erstellt.

In der WO 96/22797 A1 ist ein biokompatibler Klebstoff beschrieben, der durch mittels Laserlicht zugeführte Energie ausgehärtet wird. Wenn die den Klebstoff härtende Reaktion ausreichend weit fortgeschritten ist, ändert sich das Absorptionsvermögen des Klebstoffs, um die Absorption zu mindern.

In der WO 01/30410 A1 ist ein Material zum Kleben von Gewebe beschrieben, das durch Ausbildung kovalenter Bindungen zwischen Molekülen des Materials aushärtet. Das Material kann einen thermochromen Bestandteil umfassen.

In der US 2007/0260325 A1 sind Knochenzemente beschrieben, die thermochrome Farbstoffe zur Anzeige der Temperatur des Knochenzements enthalten.

In der EP 0 160 752 A1 ist ein Löthauteil mit einem Lot und einem Flussmittel, das bei einer kritischen Temperatur einen sichtbaren Farbwechsel zeigt, beschrieben.

Zu modernen Verfahren zählen das Verschweißen (tissue welding) und das Löten (tissue soldering) von Gewebe.

Zum Löten von Gewebe wird beispielsweise ein im Körper abbaubarer Kunststoff (auch als Biopolymer bezeichnet) als Lot verwendet. Das Lot weist eine Schmelztemperatur auf, die über der Körpertemperatur in einem Temperaturbereich liegt, in dem das Gewebe nicht oder nicht dauerhaft geschädigt wird. Zum Erwärmen des Lots wird Infrarotstrahlung verwendet, die vom Gewebe nur in geringem Maß absorbiert wird und das Gewebe deshalb nur geringfügig erwärmt. Die im Lot absorbierte Infrarotstrahlung erwärmt das Lot. Das Lot schmilzt, benetzt die zu fügenden bzw. mechanisch zu verbindenden Oberflächen und verbindet diese nach Abkühlen unter die Schmelztemperatur und Erstarren mechanisch dauerhaft, zumindest bis zur Ausbildung einer mechanisch tragfähigen Narbe.

Ähnlich wie bei anderen Lötverfahren muss die Löttemperatur genau gesteuert werden. Die Temperatur des Lots und die Temperatur der zu benetzenden Oberflächen müssen die Schmelztemperatur des Lots überschreiten, damit das Lot die zu benetzenden Oberflächen vollständig benetzt. Die Temperatur des angrenzenden Gewebes darf nicht so hoch sein, dass es dauerhaft geschädigt wird, insbesondere sich entzündet oder abstirbt.

Anders als beispielsweise beim Löten elektrischer oder elektronischer Bauteile ist der verbleibende Temperaturbereich relativ klein. Ferner hängen die absorbierte Infrarotleistung und die erzielte Temperatur von vielen Parametern ab, die nur schwer erfasst oder gesteuert werden können und stark variieren. Dazu zählen der Wasser- bzw. Feuchtegehalt des Gewebes, vor allem bei endoskopischen Anwendungen der Abstand der Infrarotquelle und die Infrarot-Intensität an der Fügestelle und die Dicke des Lots, das Wärme wesentlich schlechter leitet als in Elektrik und Elektronik verwendetes metallisches Lot. Auch ist das Schmelzen des Lots optisch oft schwer erkennbar. Wünschenswert ist deshalb eine verbesserte Steuerung oder Regelung der Temperatur beim Löten.

Eine Aufgabe der vorliegenden Erfindung besteht darin, ein verbessertes Verfahren zum Schmelzen eines Lots, ein verbessertes Lot, eine Steuerung zum Steuern eines Schmelzens eines Lots und eine Vorrichtung zum stoffschlüssigen Fügen zweier Oberflächen zu schaffen.

Diese Aufgabe wird durch die Gegenstände der unabhängigen Ansprüche gelöst.

Weiterbildungen sind in den abhängigen Ansprüchen angegeben.

Einige Ausführungsformen der vorliegenden Erfindung beruhen auf der Idee, zum stoffschlüssigen Fügen zweier Oberflächen ein nichtmetallisches oder auch ein metallisches Lot zu verwenden, das eine reversibel oder irreversibel temperaturabhängige optische Eigenschaft, insbesondere einen temperaturabhängigen Absorptionsgrad für zum Heizen des Lots verwendete elektromagnetische Strahlung, aufweist. Insbesondere nimmt der Absorptionsgrad bei einer vorbestimmten Temperatur bei oder über der Schmelztemperatur des Lots stark ab. Beispielsweise nimmt der Absorptionsgrad innerhalb eines kleinen Temperaturintervalls von 1K oder 2K oder 5K deutlich ab, beispielsweise auf zwei Drittel, die Hälfte, ein Drittel, ein Fünftel oder ein Zehntel des Werts, den er bei einer Temperatur unterhalb des Temperaturintervalls aufweist. Damit geht auch die absorbierte Leistung bei Erreichen der vorbestimmten Temperatur entsprechend zurück. Es resultiert ein verlangsamter Anstieg der Temperatur des Lots. Abhängig von der Intensität der Heizstrahlung, der Temperatur des umgebenden Gewebes und dem Wärmeverlust durch Wärmeleitung, Konvektion von Fluiden und Wärmestrahlung kann der Temperaturanstieg über der vorbestimmten Temperatur stark verlangsamt sein oder auf Null zurückgehen. Bei einer irreversiblen Reduzierung des Absorptionsgrads kann die Temperatur sogar trotz unverminderter Intensität der Heizstrahlung zurückgehen.

Die vorliegende Erfindung schafft eine passive bzw. automatische bzw. intrinsische Regelung der Temperatur des Lots. Wenn die vorbestimmte Temperatur in einem Temperaturbereich liegt, in dem Gewebe nicht oder nicht über ein vorbestimmtes Maß geschädigt wird, kann so unter bestimmten Umständen sichergestellt werden, dass beim Lötvorgang angrenzendes Gewebe nicht geschädigt wird. Ferner kann die Erfindung eine gleichmäßigere Erwärmung des Lots auch bei innerhalb des Lots deutlich variierender Intensität der Heizstrahlung ermöglichen, da Bereiche des Lots, die bereits auf die vorbestimmte Temperatur erwärmt sind, weniger Heizleistung absorbieren. Die vorliegende Erfindung ist aber auch außerhalb der Medizin einsetzbar, beispielsweise beim Löten von Kunststoffen oder Gläsern.

Bei einem Verfahren zum Schmelzen eines Lots wird das metallische oder nichtmetallische Lot mit einer temperaturabhängigen optischen Eigenschaft für elektromagnetische Strahlung mit einem vorbestimmten Spektrum im infraroten Spektralbereich bereitgestellt. Das Lot wird mit elektromagnetischer Strahlung mit dem vorbestimmten Spektrum im infraroten Spektralbereich bestrahlt. Dabei erreicht das Lot eine vorbestimmte Temperatur über seiner Schmelztemperatur, wobei sich die temperaturabhängige optische Eigenschaft des Lots bei der vorbestimmten Temperatur reversibel oder irreversibel ändert. Beim Abkühlen des Lots unter seine Schmelztemperatur erstarrt das Lot.

Bei einem Verfahren zum Kontrahieren einer Kontraktionseinrichtung wird die Kontraktionseinrichtung auf eine Oberfläche, insbesondere eine Geweheoberfläche, aufgebracht und die Kontraktionseinrichtung mit elektromagnetischer Strahlung mit einem vorbestimmten Spektrum bestrahlt. Dabei erreicht die Kontraktionseinrichtung eine vorbestimmte Temperatur und zieht sich in zumindest einer Richtung zusammen oder verändert sich geometrisch in anderer Weise. Bei der vorbestimmten Temperatur ändert sich eine temperaturabhängige optische Eigenschaft der Kontraktionseinrichtung reversibel oder irreversibel.

Dieses Verfahren ist insbesondere mit einem der oben beschriebenen Vorfahren zum stoffschlüssigen Fügen zweier Oberflächen kombinierbar, wobei beispielsweise eine der zwei zu fügenden Oberflächen eine Oberfläche der Kontraktionseinrichtung ist. Die optische Eigenschaft der Kontraktionseinrichtung ist insbesondere ihr Absortionsgrad für elektromagnetische Strahlung mit einem vorbestimmten Spektrum, wobei der Absorptionsgrad bei Erwärmung der Kontraktionseinrichtung über die vorbestimmte Temperatur abnimmt.

Die temperaturabhängige optische Eigenschaft des Lots oder der Kontraktionseinrichtung ist insbesondere der Absorptionsgrad des Lots bzw. der Kontraktionseinrichtung für elektromagnetische Strahlung mit dem vorbestimmten Spektrum, wobei der Absorptionsgrad bei Erwärmung des Lots bzw. der Kontraktionseinrichtung über die vorbestimmte Temperatur abnimmt. Bei einer human- oder veterinärmedizinischen Anwendung liegt das vorbestimmte Spektrum insbesondere im nahen Infrarotbereich (ca. 780 nm oder 750 nm bis 1,4 µm), in dem die meisten Gewebe nur wenig absorbieren.

Das Lot bzw. die Kontraktionseinrichtung wird beispielsweise innerhalb eines vorbestimmten Zeitintervalls mit einer vorbestimmten Intensität oder einer vorbestimmten Leistung bestrahlt. Die Temperatur des Lots steigt in dem vorbestimmten Zeitintervall vor Erreichen der vorbestimmten Temperatur mit einer ersten Rate und nach Erreichen der vorbestimmten Temperatur mit einer zweiten Rate, die kleiner oder wesentlich kleiner als die erste Rate ist. Beispielsweise beträgt die zweite Rate eine Hälfte, ein Drittel, ein Fünftel oder nur ein Zehntel der ersten Rate oder Null.

Vor einem der beschriebenen Verfahren kann eine Art eines Gewebes an zumindest einer der zwei zu fügenden Oberflächen erfasst werden. Es wird eine Maximaltemperatur ermittelt, bis zu der eine Schädigung von Gewebe der erfassten Art eine vorbestimmte Schwelle nicht überschreitet. Ein Lot bzw. eine Kontraktionseinrichtung wird ausgewählt und bei dem Verfahren verwendet, dessen bzw. deren optische Eigenschaft sich bei einer Temperatur ändert, die nicht größer als die ermittelte Maximaltemperatur ist. Bei der Erfassung der Art des Gewebes wird beispielsweise nach Epithelgewebe (insbesondere Oberflächen- und Drüsenepithelgewebe) und Stützgewebe (insbesondere Knochen, Knorpel und Fettgewebe), Muskelgewebe und Nervengewehe unterschieden. Als vorbestimmte Schwelle der Schädigung wird beispielsweise die Grenze zwischen einer reversiblen und einer nicht-reversiblen Schädigung verwendet. Die dazu zulässige Maximaltemperatur wird beispielsweise einer Tabelle entnommen. Die vorbestimmte Temperatur, bei der sich die temperaturabhängige optische Eigenschaft des Lots bzw. der Kontraktionseinrichtung ändert, liegt beispielsweise bei 54°C oder weniger. Es kann berücksichtigt werden, dass die Schädigung von Gewebe von nicht nur von der Temperatur sondern auch von der Zeitdauer der Einwirkung der Temperatur und vom Volumen bzw. der Masse des betroffenen Gewebes abhängt. Ferner kann berücksichtigt werden, dass das Lot bzw. die Kontraktionseinrichtung zumindest in ihrem Inneren eine höhere Temperatur erreichen als abgrenzendes Gewebe.

Bei einem der beschriebenen Verfahren kann eine Intensität oder eine Leistung einer von dem Lot bzw. der Kontraktionseinrichtung transmittierte oder reflektierten oder gestreuten elektromagnetischen Strahlung erfasst werden. Das Bestrahlen wird beispielsweise beendet, wenn die erfasste Intensität oder Leistung oder eine absolute oder relative Änderung der erfassten Intensität oder Leistung in mindestens einem vorbestimmten Spektralbereich eine vorbestimmte Schwelle über- oder unterschreitet.

Ein Lot zum stoffschlüssigen Fügen zweier Oberflächen durch Benetzen beider Oberflächen durch das geschmolzene Lot und Verbinden der Oberflächen durch das einschließend erstarrte Lot weist eine reversibel oder irreversibel temperaturabhängige optische Eigenschaft für elektromagnetische Strahlung mit einem vorbestimmten Spektrum im infraroten Spektralbereich auf, insbesondere einen temperaturabhängigen Absorptionsgrad für elektromagnetische Strahlung mit einem vorbestimmten Spektrum. Das Lot weist insbesondere ein für elektromagnetische Strahlung mit dem vorbestimmten Spektrum transparentes Polymer und zumindest entweder ein thermochromes Pigment oder einen thermochromen Farbstoff auf. Beispielsweise weist das Lot Kapseln mit einer Hülle, die für elektromagnetische Strahlung mit dem vorbestimmten Spektrum transparent ist, und mit einem von der Hülle umschlossenen thermochromen Pigment auf.

Eine Kontraktionseinrichtung weist eine reversibel oder irreversibel temperaturabhängige optische Eigenschaft auf, insbesondere einen temperaturabhängigen Absorptionsgrad für elektromagnetische Strahlung mit einem vorbestimmten Spektrum. Die Kontraktionsein-richtung weist insbesondere ein für elektromagnetische Strahlung mit dem vorbestimmten Spektrum transparentes Polymer und zumindest entweder ein thermochromes Pigment oder einen thermochromen Farbstoff auf. Beispielsweise weist die Kontraktionseinrichtung Kapseln mit einer Hülle, die für elektromagnetische Strahlung mit dem vorbestimmten Spektrum transparent ist, und mit einem von der Hülle umschlossenen thermochromen Pigment auf.

Die vorstehend beschriebenen Lote, die vorstehend beschriebenen Kontraktionseinrichtungen und die bei den vorstehend beschriebenen Verfahren verwendeten Lote und Kontraktionseinrichtungen können einen temperaturabhängigen Absorptionsgrad für elektromagnetische Strahlung mit einem vorbestimmten Spektrum im infraroten Spektralbereich, der sich bei einer ersten vorbestimmten Temperatur ändert, und ein temperaturabhängiges Absorption-, Streu- oder Fluoreszenzverhalten im sichtbaren Bereich (ca. 380 nm bis 750 nm oder 780 nm), das sich bei der ersten vorbestimmten Temperatur oder bei einer zweiten vorbestimmten Temperatur ändert, aufweisen.

Eine Steuerung zum Steuern eines Schmelzens eines Lots oder zum Kontrahieren einer Kontraktionseinrichtung ist zur Steuerung von einem der vorstehend beschriebenen Verfahren ausgebildet. Die Steuerung weist einen Eingang zum Erfassen eines Signals eines Sensors für eine von dem Lot bzw. von der Kontraktionseinrichtung transmittierte oder reflektierte oder gestreute elektromagnetische Strahlung auf.

Eine Vorrichtung zum Schmelzen eines Lots oder zum Kontrahieren einer Kontraktionseinrichtung in oder an einem menschlichen oder tierischen Körper umfasst die vorstehend beschriebene Steuerung und eine Lichtquelle zum Bestrahlen des Lots bzw. der Kontraktionseinrichtung mit elektromagnetischer Strahlung mit dem vorbestimmten Spektrum. Ferner umfasst die Vorrichtung einen Sensor zum Erfassen von elektromagnetischer Strahlung, die von dem Lot bzw. der Kontraktionseinrichtung transmittiert oder reflektiert oder gestreut ist.

Die Erfindung ist in den Ansprüchen definiert.

### Kurzbeschreibung der Figuren

Nachfolgend werden Ausführungsformen anhand der beigefügten Figuren näher erläutert. Es zeigen:
- Figur 1: eine schematische Darstellung einer Vorrichtung zum stoffschlüssigen Fügen zweier Oberflächen;
- Figur 2: eine schematische Darstellung zweier stoffschlüssig zu fügender Oberflächen;
- Figur 3: ein schematisches Diagramm einer Zeitabhängigkeit einer Temperatur;
- Figur 4: eine schematische Darstellung einer Kapsel mit einem thermochromen Pig- ment;
- Figur 5: eine schematische Darstellung einer Kontraktionseinrichtung;
- Figur 6: eine schematische Darstellung der Kontraktionseinrichtung aus Figur 5 in kontrahierten Zustand;
- Figur 7: ein schematisches Flussdiagramm eines Verfahrens zum stoffschlüssigen Fügen zweier Oberflächen;
- Figur 8: ein schematisches Flussdiagramm eines Verfahrens zum Schrumpfen einer Kontraktionseinrichtung.

### Beschreibung der Ausführungsformen

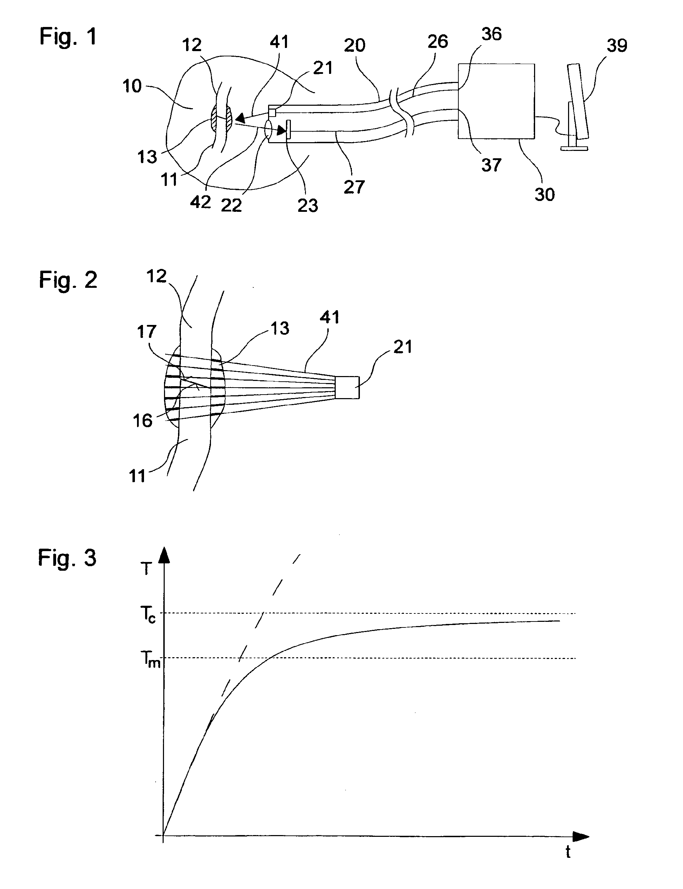

Figur 1 zeigt eine schematische Darstellung eines Querschnitts einer Vorrichtung zum stoffschlüssigen Fügen zweier Oberflächen, insbesondere zum stoffschlüssigen Fügen zweier Oberflächen in einem menschlichen oder tierischen Körper. In einem Hohlraum 10 sind ein erstes Teil 11 und ein zweites Teil 12 mittels eines Lots 13 zu fügen. Die Vorrichtung umfasst ein Endoskop 20, dessen distales Ende in den Hohlraum 10 eingeführt werden kann. Am distalen Ende des Endoskops 20 sind eine Lichtquelle 21, ein Objektiv 22 und ein Bildsensor 23 angeordnet. Die Lichtquelle 21 ist mit einer Beleuchtungsleitung 26 verbunden. Der Bildsensor 23 ist mit einer Bildleitung 27 verbunden. Die Vorrichtung umfasst ferner eine Steuerung 30 mit einem Beleuchtungsausgang 36, der über die Beleuchtungsleitung 26 mit der Lichtquelle 21 gekoppelt ist, und einen Bildsignaleingang 37, der über die Bildleitung 27 mit dem Bildsensor 23 gekoppelt ist. Die Vorrichtung umfasst ferner eine Benutzerschnittstelle 39, beispielsweise einen Bildschirm und/oder eine Tastatur oder andere Eingabeeinrichtung, die mit der Steuerung 30 gekoppelt ist.

Die Lichtquelle 21 ist ausgebildet, um Heizstrahlung 41 zu emittieren. Dazu umfasst die Lichtquelle 21 beispielsweise eine Leuchtdiode oder einen Halbleiterlaser. Die Heizstrahlung umfasst beispielsweise ein vorbestimmtes Spektrum im nahen Infrarot (Wellenlängen von ca. 780 nm oder 750 nm bis 1,4 µm), im mittleren Infrarot (1,4 µm bis 3 µm) oder im fernen Infrarot (3 µm bis 1 mm). Da elektromagnetische Strahlung im nahen Infrarot von menschlichem oder tierischem Gewebe nur geringfügig absorbiert wird, ist dieser Spektralbereich besonders geeignet.

Das Objektiv 22 ist angeordnet, ausgebildet und vorgesehen, um gestreute oder reflektierte Heizstrahlung 42 auf den Bildsensor 23 zu fokussieren. Der Bildsensor 23 kann so ein Bild eines Gegenstands, der von der Lichtquelle 21 mit Heizstrahlung 41 bestrahlt wird, erfassen. Beim in Figur 1 dargestellten Beispiel erfasst der Bildsensor 23 ein Bild des ersten Teils 11, des zweiten Teils 12 und des Lots 13. Das vom Bildsensor 23 erfasste Bild wird beispielsweise nach Aufbereitung durch die Steuerung 30 an der Benutzerschnittstelle 39 dargestellt.

Die Lichtquelle 21 kann statt am distalen Ende des Endoskops 20 am proximalen Ende des Endoskops 20 oder in der Steuerung 30 angeordnet sein. In diesem Fall ist die Lichtquelle 21 über einen Lichtwellenleiter mit dem distalen Ende des Endoskops 20 gekoppelt. Der Bildsensor 23 kann statt am distalen Ende des Endoskops 20 am proximalen Ende des Endoskops 20 oder in der Steuerung 30 angeordnet sein. In diesem Fall ist der Bildsensor 23 über einen Bildleiter, insbesondere ein geordnetes Bündel von Lichtwellenleitern, mit dem distalen Ende des Endoskops 20 gekoppelt. Das proximale Ende des Endoskops 20 kann, wie in Figur 1 dargestellt, unmittelbar mit der Steuerung 30 verbunden oder über ein Kabel oder eine Leitung mit der Steuerung 30 gekoppelt sein.

Anstelle des Bildsensors 23 kann ein nicht-ortsempfindlicher Sensor für gestreute oder reflektierte Heizstrahlung vorgesehen sein. Der Sensor oder Bildsensor 23 kann neben Empfindlichkeit für gestreute oder reflektierte Heizstrahlung auch eine Empfindlichkeit für elektromagnetische Strahlung mit anderen Wellenlängen aufweisen. Beispielsweise ist der Sensor oder Bildsensor 23 empfindlich für Wellenlängen im nahen Infrarot und im sichtbaren Bereich. Die Lichtquelle 21 kann ausgebildet sein, um zusätzlich zur Heizstrahlung 41 elektromagnetische Strahlung mit anderen Wellenlängen zu emittieren. Beispielsweise ist die Lichtquelle ausgebildet, um Licht im nahen Infrarot und im sichtbaren Bereich zu emittieren.

Die in Figur 1 dargestellte Vorrichtung ist ausgebildet, um eine Ausrichtung des distalen Endes des Endoskops 20 auf die Fügestelle zwischen dem ersten Teil 11 und dem zweiten Teil 12 und das Lot 13, ein Bestrahlen des Lots 13 mit der Heizstrahlung 41 und ein Beobachten des Lots 13 über den Bildsensor 23 zu ermöglichen. Der dabei ablaufende Lötvorgang wird nachfolgend mit Bezug auf die Figuren 2 bis 4 näher beschrieben.

Figur 2 zeigt eine schematische Darstellung eines Ausschnitts aus Figur 1 mit weiteren Details. Dargestellt sind das erste Teil 11, das zweite Teil 12, das Lot 13, die Lichtquelle 21 und die von der Lichtquelle 21 emittierte Heizstrahlung 41. Das erste Teil 11 und das zweite Teil 12 sind beispielsweise Enden zweier Blutgefäße oder Enden zweier Sehnen, die miteinander gefügt bzw. mechanisch verbunden werden sollen. Insbesondere sollen eine Oberfläche 16 des ersten Teils 11 und eine Oberfläche 17 des zweiten Teils 12 miteinander verbunden werden. Die Bezugszeichen 16 und 17 weisen in Figur 2 insbesondere auf die einander gegenüberliegenden Stirnflächen des ersten Teils 11 und des zweiten Teils 12. Tatsächlich erfolgt die mechanische Verbindung aber vor allem auch über angrenzende Abschnitte der Mantelflächen des ersten Teils 11 und des zweiten Teils 12, die von dem Lot 13 benetzt und nach dessen Erstarren mechanisch verbunden werden. Mit den zu fügenden Oberflächen 16, 17 sind deshalb im Folgenden neben den Stirnflächen auch die angrenzenden Mantelflächen des ersten Teils 11 und des zweiten Teils 12 gemeint. Allgemein bezieht sich der Begriff der zu fügenden Oberflächen auf die gesamten mit Lot zu benetzenden Oberflächen.

Bei der Darstellung in Figur 2 benetzt das Lot 13 bereits die Oberflächen der Teile 11, 12. Dies setzt in der Regel voraus, dass das Lot 13 aufgeschmolzen ist oder bereits aufgeschmolzen war.

Die von der Lichtquelle 21 ausgehende Heizstrahlung 41 ist in Figur 2 ungeachtet ihrer Natur als elektromagnetische Welle und als Vielzahl von Photonen und ungeachtet ihrer kontinuierlichen Intensitätsverteilung durch mehrere einzelne gerade Strahlen dargestellt. Auch die Brechung der Heizstrahlung an Oberflächen des Lots 13 und der Teile 11, 12 ist vereinfachend nicht dargestellt. Die die Heizstrahlung 41 repräsentierenden Strahlen sind dort als dünne Linien dargestellt, wo die Heizstrahlung 41 nicht oder fast nicht absorbiert wird, insbesondere in dem mit Luft, Stickstoff oder einem anderen Fluid gefüllten Hohlraum und in den Teilen 11, 12. Dazu ist beispielsweise die Wellenlänge der Heizstrahlung 41 so ausgewählt, dass sie in den Teilen 11, 12 nicht oder fast nicht absorbiert wird. In Bereichen, in denen die Heizstrahlung 41 stark absorbiert wird, sind die die Heizstrahlung repräsentierenden Strahlen als breite Linien dargestellt. Dies ist insbesondere innerhalb des Lots 13 der Fall. Dazu ist das Lot 13 so ausgewählt, dass es die Heizstrahlung 41 stark absorbiert.

Das Lot 13 umfasst beispielsweise als Matrix einen für die Heizstrahlung 41 im Wesentlichen transparenten Kunststoff mit einem Schmelzpunkt in einem Bereich zwischen einigen K über der Körpertemperatur des menschlichen oder tierischen Körpers bis 54°C oder einige K darunter. Vorteilhaft ist die Verwendung eines für den menschlichen bzw. tierischen Körper nicht-toxischen Kunststoffs. Vorteilhaft ist ferner eine Abbaubarkeit bzw. Resorbierbarkeit des Kunststoffs. Der Kunststoff ist beispielsweise Polydiacetylen oder ein von Polydiacetylen abgeleitetes Polymer. Das Lot 13 weist ferner ein Thermochrom (thermochromer Farbstoff oder thermochromes Pigment) auf, das die Heizstrahlung 41 absorbiert, wenn seine Temperatur unter oder nicht über einer vorbestimmten Temperatur liegt. Die thermochrome Eigenschaft kann eine intrinsische Eigenschaft des Farbstoffs oder Pigments sein oder von einer Wechselwirkung des Farbstoffs bzw. Pigments mit dem Polymer oder einem anderen Bestandteil des Lots 13 herrühren.

Da die Heizstrahlung 41 im Wesentlichen nur im Lot 13 absorbiert wird oder vom Lot 13 zumindest deutlich stärker absorbiert wird als von den Teilen 11, 12, erwärmt die Lichtquelle 21 über die Heizstrahlung 41 unmittelbar ausschließlich oder fast ausschließlich das Lot 13 oder zumindest das Lot 13 stärker oder deutlich stärker als die Teile 11, 12 oder andere Teile des menschlichen oder tierischen Körpers. Mittelbar werden durch Wärmeleitung, Konvektion und vom erwärmten Lot 13 ausgehende Wärmestrahlung auch die Teile 11, 12 und andere Teile des menschlichen oder tierischen Körpers erwärmt, erreichen dabei jedoch in der Regel nicht die Temperatur des Lots 13.

Die von der Lichtquelle 21 erzeugte Heizstrahlung 41 kann, wie in Figur 2, divergent sein. Die auf das Lot 13 treffende Heizstrahlung 41 kann jedoch beispielsweise bei Verwendung eines Lasers als Lichtquelle 21 und/oder eines Kollimators eine sehr geringe Divergenz aufweisen oder auf das Lot 13 oder Teilbereiche des Lots 13 fokussiert sein.

Figur 3 zeigt ein schematisches Diagramm der Abhängigkeit der Temperatur T des Lots 13 von der Zeit t. Der Abszisse ist die Zeit t zugeordnet, der Ordinate ist die Temperatur T zugeordnet. Eingezeichnet ist eine Minimaltemperatur Tₘ, die das Lot 13 erreichen muss, um vollständig aufzuschmelzen und die zu fügenden Oberflächen zu benetzen. Die Minimaltemperatur Tₘ ist beispielsweise durch die Schmelztemperatur des Lots 13 zuzüglich einer Sicherheitsmarge gegeben. Ferner ist in Figur 3 eine kritische Temperatur T_{c} eingezeichnet, die das Lot 13 nicht überschreiten darf, um eine thermische Schädigung von angrenzendem Gewebe (insbesondere der Teile 11, 12) zu vermeiden.

Ferner ist in Figur 3 als unterbrochene Linie der Temperaturverlauf im Falle einer herkömmlichen, nicht-thermochromen Absorption dargestellt. Die Temperatur des Lots steigt bei konstanter Intensität der Heizstrahlung 41 zunächst linear an. Die Geschwindigkeit des Anstiegs ist durch die Intensität bzw. Leistung der Heizstrahlung 41, die Absorptionskonstante des Lots 13 für die Heizstrahlung 41 und die Wärmekapazität des Lots 13 gegeben. Mit zunehmender Temperatur ist eine leichte Abflachung zu beobachten, die von Wärmeverlusten des Lots 13 an seine Umgebung durch Wärmeleitung, Konvektion und Wärmestrahlung herrührt.

Als durchgezogene Linie ist die Zeitabhängigkeit der Temperatur für ein Lot 13 mit einem thermochromen Absorptionsverhalten dargestellt. Die Absorptionskonstante des Lots 13 für die Heizstrahlung 41 nimmt bei einer vorbestimmten Temperatur deutlich ab. Beispielsweise nimmt die Absorptionskonstante innerhalb eines Temperaturintervalls von 1K, 2K, 5K oder 10K auf die Hälfte, ein Drittel, ein Fünftel oder ein Zehntel ab. Auch eine sprunghafte bzw. nicht-kontinuierliche Abnahme ist möglich, beispielsweise bedingt durch einen Phasenübergang erster Ordnung oder ähnlich einem Phasenübergang erster Ordnung.

Die durchgezogene Linie in Figur 3 zeigt eine Zeitabhängigkeit der Temperatur, die herrührt von einer Abnahme der Absorption der Heizstrahlung 41 durch das Lot 13 bereits unterhalb der Mindesttemperatur Tₘ, wobei die Absorption bei Temperaturen zwischen der Mindesttemperatur Tₘ und der kritischen Temperatur T_{c} bereits stark zurückgegangen ist. Dieses Absorptionsverhalten kann von einer nicht-sprunghaften Temperaturabhängigkeit des Absorptionsverhaltens des Lots 13 und/oder von der Geometrie des Volumens, das vom Lot 13 erfüllt wird, herrühren.

Es resultiert eine Zeitabhängigkeit der Temperatur T des Lots 13 mit einem raschen Temperaturanstieg für kleine Temperaturen, einer bei der Mindesttemperatur Tₘ bereits deutlichen Abflachung des Temperaturanstiegs und einer nachfolgenden asymptotischen Annäherung der Temperatur T an eine Temperatur unterhalb der kritischen Temperatur T_{c}. Mit diesem Temperaturverlauf ist sichergestellt, dass, eine ausreichend lange Bestrahlungsdauer vorausgesetzt, das Lot 13 die Mindesttemperatur Tₘ überschreitet und somit vollständig aufschmilzt und die zu fügenden Oberflächen 16, 17 benetzt. Gleichzeitig ist sichergestellt, dass die Temperatur T des Lots 13 die kritische Temperatur T_{c} auch bei längerer Bestrahlung nicht überschreitet und an das Lot 13 angrenzendes Gewebe nicht oder nicht über ein vorbestimmtes Maß hinaus geschädigt wird.

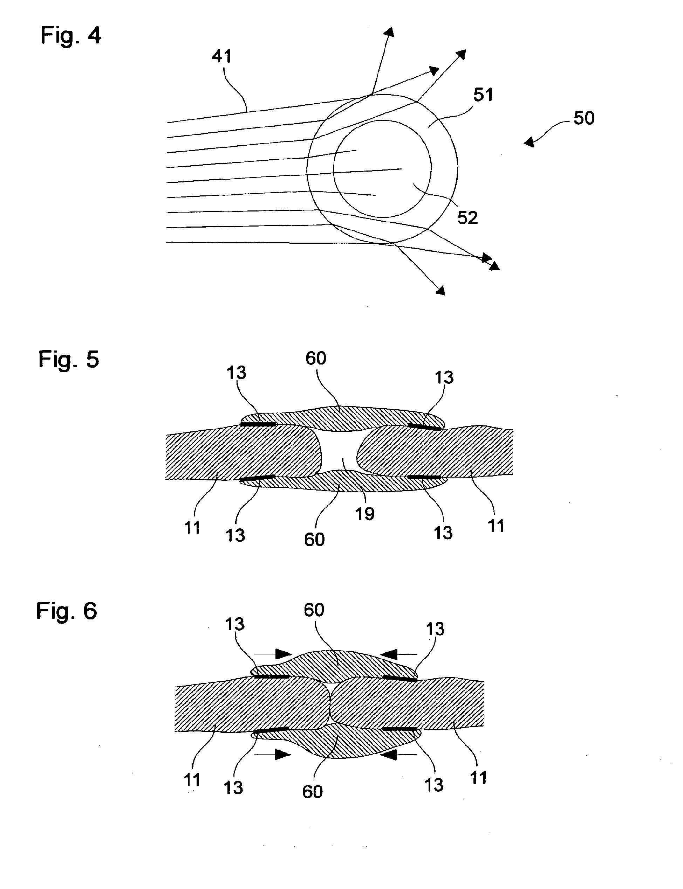

Das Lot 13 kann, wie erwähnt, einen thermochromen Farbstoff oder auch thermochromes Pigment in einer an sich für die Heizstrahlung 41 transparenten Matrix aufweisen. Ein thermochromer Farbstoff oder ein thermochromes Pigment - beispielsweise ein Flüssigkristall - kann in Form mikroskopischer Kapseln in der Matrix des Lots 13 dispergiert sein. Figur 4 zeigt eine schematische Darstellung eines Querschnitts durch eine solche Kapsel 50 und Heizstrahlung 41, die auf die Kapsel 50 trifft. Die Kapsel 50 weist eine für die Heizstrahlung 41 transparente oder im Wesentlichen transparente Hülle 51 auf, die das Thermochrom 52 umschließt. Ein Umschließen des Thermochroms 52 durch eine Hülle 51 ist beispielsweise vorteilhaft, wenn das Thermochrom eine toxische Wirkung auf den menschlichen oder tierischen Körper aufweist oder mit der Matrix des Lots 13 nicht mischbar ist. Die Kapsel weist beispielsweise einen Durchmesser zwischen 1 µm und 100 µm auf, insbesondere einen Durchmesser von 5 µm. Der gesamte durch die Hülle 51 umschlossene Raum enthält beispielsweise einen unverdünnten oder in einem geeigneten Lösungsmittel verdünnten oder in ein Gel oder eine andere Matrix eingebetteten Farbstoff, ein oder mehrere in eine feste Matrix eingebettete Partikel eines Pigments oder ein oder mehrere Partikel eines Pigments in einer Flüssigkeit. Alternativ umschließt die Hülle 51 beispielsweise ein einziges Partikel eines Pigments.

In Figur 4 ist eine Hülle 51 dargestellt, deren Brechungsindex kleiner ist als der Brechungsindex der die Kapsel 50 umgebenden Matrix. Es resultiert eine Brechung der Heizstrahlung 41 an der äußeren Oberfläche der Hülle 51, am Rand auch eine Totalreflexion. Dies hat zwei Folgen. Einerseits trifft weniger Heizstrahlung auf den Thermochrom 52, andererseits streut die Kapsel 50 die Heizstrahlung 41 auch bei Temperaturen über der vorbestimmten Temperatur, bei denen der Thermochrom 52 für die Heizstrahlung 41 transparent oder im Wesentlichen transparent ist. Die Streuung verlängert den optischen Weg der Heizstrahlung 41 im Lot 13 und vergrößert so ihre Absorption. Besser als die in Figur 4 dargestellte Verwendung einer Hülle 51 mit einem Brechungsindex kleiner als der Brechungsindex der umgebenden Matrix ist daher eine Verwendung einer Hülle 51 mit einem Brechungsindex, der größer ist als der Brechungsindex der umgebenden Matrix. Besonders vorteilhaft ist eine Hülle 51, deren Brechungsindex dem Brechungsindex der umgebenden Matrix entspricht. Soweit der Brechungsindex des von der Hülle 51 umschlossenen Thermochroms 52 frei einstellbar ist, sollte dieser zumindest bei Temperaturen über der vorbestimmten Temperatur möglichst genau auf den Brechungsindex der Hülle 51 und den Brechungsindex der die Kapsel 50 umgebenden Matrix eingestellt sein. Der Brechungsindex der Hülle 51 oder der Matrix ist beispielsweise durch Beimengung von löslichen, unlöslichen oder schwer löslichen Zuckern einstellbar.

Die Figuren 5 und 6 zeigen schematische Darstellungen eines Querschnitts durch einen Teil 11 eines menschlichen oder tierischen Körpers und eine Kontraktionseinrichtung 60. Das Teil 11 ist flächig, weist also in einer Dimension eine viel kleinere Ausdehnung auf als in den beiden anderen Dimensionen. Das Teil 11 ist beispielsweise eine Wand eines Magens oder eines Darms in einem menschlichen oder tierischen Körper. Die in den Figuren 5 und 6 dargestellten Schnittebenen liegen jeweils senkrecht zum Teil 11 bzw. zu seiner lokalen Tangentialebene.

Das Teil 11 weist ein Durchgangsloch bzw. eine Öffnung 19 auf, wie sie beispielsweise im Rahmen der NOTES (Natural Orifice Transluminal Endoscopic Surgery) in einer Magenwand oder einer Darmwand erzeugt und nach Ende des Eingriffs wieder verschlossen wird. Die Öffnung 19 wird durch zwei auf den gegenüberliegenden Seiten des Teils 11 angeordnete Kontraktionseinrichtungen 60 verschlossen. Jede der beiden Kontraktionseinrichtungen 60 ist beispielsweise tellerförmig oder kreisförmig oder elliptisch oder rechteckig und weist ein elastisches, insbesondere biokompatibles Material auf. Nahe seinem äußeren Rand wird jede Kontraktionseinrichtung 60 mittels Lot 13 mit dem Teil 11 gefügt bzw. mechanisch verbunden. Dies erfolgt beispielsweise ähnlich wie oben anhand der Figuren 1 bis 4 beschrieben, indem das Lot 13 mittels Heizstrahlung auf eine Temperatur über seinem Schmelzpunkt erwärmt und so geschmolzen wird und nach dem Erstarren eine stoffschlüssige Verbindung mit dem Teil 11 und der Kontraktionseinrichtung 60 bildet.

Figur 5 zeigt die Kontraktionseinrichtungen 60 nach ihrer stoffschlüssigen Verbindung mit dem Teil 11 in einem expandierten Zustand. Figur 6 zeigt die Kontraktionseinrichtungen 60 in einem kontrahierten Zustand. Die Kontraktionseinrichtungen 60 werden nach der stoffschlüssigen Verbindung mit dem Teil 11 von dem in Figur 5 gezeigten expandierten Zustand in den in Figur 6 gezeigten kontrahierten Zustand überführt. Die Pfeile in Figur 6 deuten an, dass dabei die lateralen Abmessungen der Kontraktionseinrichtungen 60 verringert und das Teil 11 zusammengezogen wird. Dabei wird die Öffnung 19 im Teil 11 geschlossen oder zumindest deutlich verkleinert. Dies ermöglicht eine deutlich beschleunigte Vemarbung der Öffnung 19. Die Kontraktionseinrichtungen 60 können ein Material aufweisen, das vom menschlichen bzw. tierischen Körper abgebaut bzw. resorbiert werden kann, so dass nach Vemarbung der Öffnung 19 die Kontraktionseinrichtungen 60 langsam verschwinden.

Die laterale Kontraktion der Kontraktionseinrichtung 60 beruht beispielsweise auf einem ähnlichen Prinzip wie die Kontraktion von Schrumpfschläuchen, die in der Elektrotechnik und Elektronik zur elektrischen Isolation verwendet werden, oder wie die Kontraktion von Schrumpffolien, wie sie zur Verpackung von Gütern aller Art oder auch im Leichtflugzeugbau und im Modellbau zum Bespannen von Tragflächen verwendet werden. Insbesondere kann die Kontraktionseinrichtung 60 nach der Herstellung und der Vernetzung von Molekülketten ihres Materials im kontrahierten Zustand bei einer erhöhten Temperatur gedehnt und dann im gedehnten Zustand abgekühlt werden. Der gedehnte Zustand wird dabei fixiert oder eingefroren. Bei erneuter Erwärmung über eine vorbestimmte Temperatur geht die Kontraktionseinrichtung 60 dann in den kontrahierten Zustand über, den sie bei der Vernetzung der Molekülketten ihres Materials einnahm.

Die laterale Kontraktion der Kontraktionseinrichtungen 60 kann beispielsweise thermisch (ähnlich wie bei Schrumpfschläuchen oder Schrumpffolien) oder photochemisch ausgelöst werden. Dazu weisen die Kontraktionseinrichtungen 60 beispielsweise den gleichen Farbstoff oder das gleiche Pigment auf wie das Lot 13, um Heizstrahlung zu absorbieren. Alternativ weisen die Kontraktionseinrichtungen 60 beispielsweise andere Farbstoffe oder Pigmente auf, die bei anderen Wellenlängen absorbieren. Dadurch können der Lötvorgang und der Schrumpf- bzw. Kontraktionsvorgang unabhängig voneinander gesteuert werden.

In jedem Fall ist eine thermochrome Eigenschaft der Kontraktionseinrichtungen 60 vorteilhaft, insbesondere die Verwendung thermochromer Farbstoffe oder Pigmente in den Kontraktionseinrichtungen 60. Dadurch können, ähnlich wie oben anhand der Figuren 1 bis 4 beschrieben, die in den Kontraktionseinrichtungen 60 und in angrenzendem Gewebe erzeugten Temperaturen beschränkt werden, um eine Überhitzung und Schädigung von Gewebe zu verhindern.

Alternativ zu einer durch Heizstrahlung hervorgerufenen thermischen Schrumpfung der Kontraktionseinrichtungen 60 ist auch eine Schrumpfung mittels anderer Mechanismen möglich. Ferner können die Kontraktionseinrichtungen 60 statt durch Lot 13 auch auf andere Weise mit dem Teil 11 gefügt werden. Beispielsweise weisen die Kontraktionseinrichtungen 60 ein saugfähiges Material, das sich aufgrund seiner Saugfähigkeit bzw. der dieser zugrunde liegenden Kapillarwirkung an die Oberflächen des Teils 11 anlegt und an diesem haftet.

Abweichend von den Darstellungen in den Figuren 5 und 6 können ferner beide Kontraktionseinrichtungen 60 über einen Steg aus dem gleichen Material und/oder einen Stift aus einem anderen Material miteinander verbunden sein. Der Steg und/oder der Stift können verwendet werden, um die Kontraktionseinrichtungen 60 an der Öffnung anzuordnen. Dabei wird beispielsweise zunächst ein Rohr in die Öffnung 19 eingeführt, durch das die über den Steg und/oder den Stift miteinander verbundenen und gefalteten Kontraktionseinrichtungen 60 in die Öffnung 19 eingeführt werden. Das Rohr wird dann so zurückgezogen, dass sich je eine der beiden Kontraktionseinrichtungen 60 an jeder Seite des Teils 11 entfaltet und, wie in Figur 5 dargestellt, an das Teil 11 anlegt. Der Stift kann gegebenenfalls vor oder nach dem Schrumpfen bzw. Kontrahieren der Kontraktionseinrichtungen 60 beispielsweise durch ruckartiges Ziehen, Drehen oder Schrauben gelöst und aus der Öffnung 19 entfernt werden.

Anstelle einer punktförmigen Öffnung können eine oder zwei Kontraktionseinrichtungen auch linienförmige Öffnungen in einem Teil eines menschlichen oder tierischen Körpers verschließen. Die Darstellung eines Querschnitts vor und nach dem Kontrahieren der Kontraktionseinrichtungen entspricht in diesem Fall weitgehend den Darstellungen in den Figuren 5 und 6.

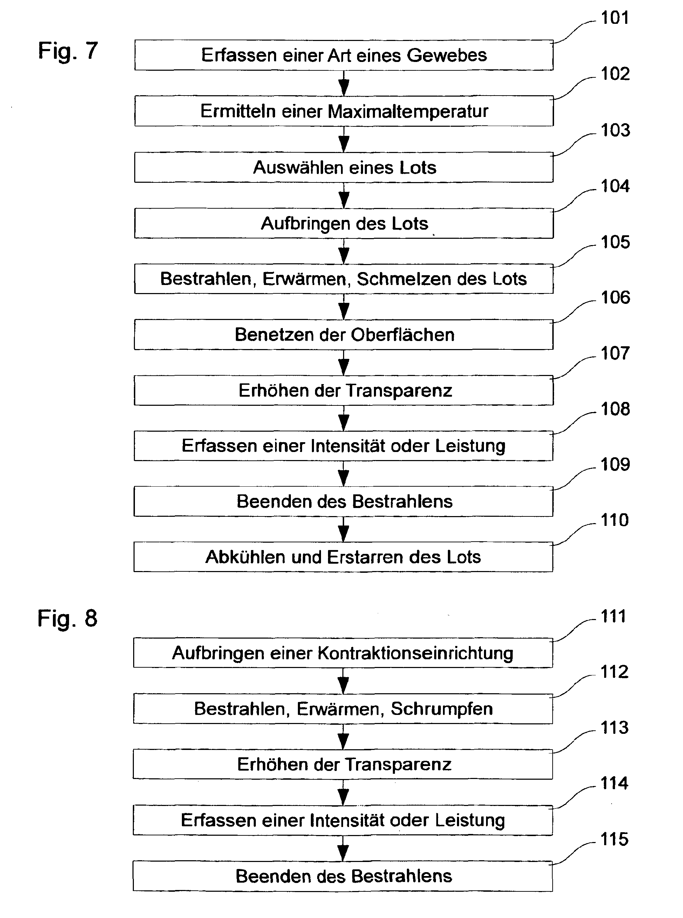

Die Figuren 7 und 8 zeigen schematische Flussdiagramme eines Verfahrens zum stoffschlüssigen Fügen zweier Oberflächen bzw. eines Verfahrens zum Verschließen einer Öffnung. Obwohl beide Verfahren auch mit Vorrichtungen und Einrichtungen sowie an Körperteilen oder Werkstücken verwendbar sind, die sich von den oben anhand der Figuren 1 bis 6 dargestellten unterscheiden, werden nachfolgend beispielhaft Bezugszeichen aus den Figuren 1 bis 6 verwendet, um ein Verständnis zu erleichtern. Die nachfolgend anhand der Figuren 7 und 8 dargestellten Verfahren können miteinander kombiniert, insbesondere nacheinander oder teilweise gleichzeitig ausgeführt werden. Ferner können die beiden Verfahren unabhängig voneinander ausgeführt werden. Einige der beschriebenen Schritte sind optional, worauf unten teilweise nochmals explizit hingewiesen wird.

Figur 7 bezieht sich auf ein Verfahren zum Löten bzw. zum stoffschlüssigen Fügen zweier Oberflächen mittels eines Lots. Bei einem ersten Schritt 101 wird eine Art eines Gewebes an zumindest einer von zwei stoffschlüssig zu fügenden Oberflächen erfasst. Die Art des Gewebes resultiert beispielsweise bereits aus der Operationsplanung. Bei einem zweiten Schritt 102 wird eine Maximaltemperatur ermittelt, bis zu der eine Schädigung von Gewebe der erfassten Art eine vorbestimmte Schwelle nicht überschreitet. Der zweite Schritt 102 kann durch Nachschlagen in einer Tabelle erfolgen. Bei einem dritten Schritt 103 wird ein Lot ausgewählt, dessen Absorptionskonstante mit Bezug auf das Spektrum einer zum Erwärmen des Lots vorgesehenen Heizstrahlung bei Überschreiten einer Temperatur deutlich abnimmt, die geringer oder nicht größer als die ermittelte Maximaltemperatur ist. Auf den ersten Schritt 101, den zweiten Schritt 102 und den dritten Schritt 103 kann beispielsweise verzichtet werden, wenn nur ein Lot zur Verfügung steht, das für mehrere oder alle Gewebe geeignet ist.

Bei einem vierten Schritt 104 wird das Lot 13 auf zumindest eine der zwei stoffschlüssig zu fügenden Oberflächen aufgebracht. Bei einem fünften Schritt 105 wird das Lot 13 mit Heizstrahlung 41 bestrahlt. Durch Absorption der Heizstrahlung 41 wird das Lot 13 bis über seine Schmelztemperatur erwärmt und so geschmolzen. Bei einem sechsten Schritt 106 benetzt das geschmolzene Lot 13 die zu fügenden Oberflächen 16, 17. Bei einem siebten Schritt 107 erhöht sich die Transparenz des Lots 13. Alternativ vermindert sich der Absorptionsgrad oder kann sich eine andere optische Eigenschaft des Lots 13 ändern. Der siebte Schritt 107 läuft insbesondere schlagartig oder kontinuierlich bei Erreichen einer vorbestimmten Temperatur des Lots 13 oder innerhalb eines kleinen Temperaturintervalls um diese vorbestimmte Temperatur ab.

Bei einem optionalen achten Schritt 108 wird die Intensität oder Leistung einer von dem Lot 13 transmittierten oder reflektierten oder gestreuten elektromagnetischen Strahlung erfasst. Wenn eine absolute oder relative Änderung der erfassten Intensität bzw. Leistung eine vorbestimmte Schwelle über- oder unterschreitet, wird das Bestrahlen des Lots 13 mit Heizstrahlung 41 beendet oder die Leistung oder Intensität der Heizstrahlung 41 reduziert.

Bei einem neunten Schritt 109 wird das Bestrahlen des Lots 13 mit Heizstrahlung 41 beendet. Der neunte Schritt 109 kann nach Ablauf eines vorbestimmten Zeitintervalls und/oder, wie bereits beschrieben, gegebenenfalls aufgrund der im optionalen achten Schritt 108 erfassten Intensität oder Leistung gesteuert werden.

Bei einem zehnten Schritt 110 kühlt das Lot 13 nach Beenden des Bestrahlens oder bereits nach Verringerung seines Absorptionsgrads für die Heizstrahlung 41 ab, erstarrt und verbindet die zu fügenden Oberflächen 16, 17 stoffschlüssig.

Figur 8 bezieht sich auf ein Verfahren zum Schrumpfen bzw. Kontrahieren einer Kontraktionseinrichtung 60. Vor den nachfolgend dargestellten Schritten können den oben anhand der Figur 7 dargestellten Schritten 101 bis 103 entsprechende Schritte ausgeführt werden, um eine geeignete Kontraktionseinrichtung auszuwählen.

Bei einem ersten Schritt 111 wird die Kontraktionseinrichtung 60 auf eine Oberfläche eines Teils 11 eines menschlichen oder tierischen Körpers aufgebracht. Dies kann mit einem Verfahren erfolgen, wie es oben anhand der Figur 7 dargestellt wurde. Alternativ kann die Kontraktionseinrichtung jedoch auch auf das Teil 11 geklebt, geschweißt oder auf andere Weise mit dem Teil 11 verbunden werden. Beispielweise kann das Teil 11 durch Kapillarkräfte bzw. einen Kapillarsog an das Teil 11 gebunden werden.

Bei einem zweiten Schritt 112 wird die Kontraktionseinrichtung bestrahlt und durch dabei absorbierte Leistung erwärmt. Aufgrund der Erwärmung schrumpft bzw. kontrahiert die Kontraktionseinrichtung 60. Dabei wird beispielsweise, wie oben anhand der Figuren 5 und 6 dargestellt, eine Öffnung 19 geschlossen. Die Kontraktion der Kontraktionseinrichtung 60 erfolgt beispielsweise ab einer ersten vorbestimmten Temperatur oder innerhalb eines Temperaturintervalls. Bei einem dritten Schritt 113 erhöht sich die Transparenz bzw. verringert sich die Absorptionskonstante der Kontraktionseinrichtung für elektromagnetische Strahlung mit einem vorbestimmten Spektrum. Wenn die Erwärmung der Kontraktionseinrichtung mittels elektromagnetischer Strahlung mit dem vorbestimmten Spektrum erfolgt, resultiert aus der verringerten Absorptionskonstante eine entsprechende Verminderung der absorbierten Leistung. Dadurch wird der Temperaturanstieg der Kontraktionseinrichtung 60 vermindert oder er geht auf Null zurück oder die Temperatur der Kontraktionseinrichtung 60 nimmt ab.

Bei einem optionalen vierten Schritt 114 wird eine Intensität oder eine Leistung einer von der Kontraktionseinrichtung transmittierten oder reflektierten oder gestreuten elektromagnetischen Strahlung erfasst.

Bei einem fünften Schritt 115 wird das Bestrahlen der Kontraktionseinrichtung 60 beendet oder die Intensität oder Leistung der Bestrahlung reduziert. Der fünfte Schritt kann zeitgesteuert bzw. nach Ablauf eines vorbestimmten Zeitintervalls oder gegebenenfalls in Abhängigkeit von der beim optionalen vierten Schritt 114 erfassten Intensität bzw. Leistung gesteuert werden. Insbesondere kann das Bestrahlen der Kontraktionseinrichtung 60 beendet werden, wenn die erfasste Intensität oder Leistung oder eine absolute oder relative Änderung der erfassten Intensität oder Leistung eine vorbestimmte Schwelle über- oder unterschreitet.

Sowohl das bei den oben anhand der Figuren 1 bis 7 beschriebenen Ausführungsbeispielen verwendete Lot 13 als auch die Kontraktionseinrichtungen 60 der oben anhand der Figuren 5, 6 und 8 dargestellten Beispiele können neben einem temperaturabhängigen Absorptionsgrad für elektromagnetische Strahlung mit einem ersten vorbestimmten Spektrum (beispielsweise im nahen Infrarot) ein temperaturabhängiges Absorptions-, Streu- oder Fluoreszenzverhalten in einem zweiten vorbestimmten Spektrum (beispielsweise im sichtbaren Bereich) aufweisen. Der temperaturabhängige Absorptionsgrad für elektromagnetische Strahlung mit dem ersten vorbestimmten Spektrum und das temperaturabhängige Absorptions-, Streu- oder Fluoreszenzverhalten in einem zweiten vorbestimmten Spektralbereich können sich bei der gleichen vorbestimmten Temperatur oder bei zwei unterschiedlichen vorbestimmten Temperaturen ändern. Die Abnahme des Absorptionsgrads bei Überschreiten einer ersten vorbestimmten Temperatur hat eine automatische bzw. intrinsische bzw. selbsttätige Regulierung der Temperatur des Lots 13 bzw. der Kontraktionseinrichtung 60 zur Folge. Die Änderung des Absorptions-, Streu- oder Fluoreszenzverhaltens ist geeignet, das Erreichen der zweiten vorbestimmten Temperatur für das menschliche Auge sichtbar oder mit einem entsprechenden Sensor erfassbar zu signalisieren.

Die Erfindung eignet sich zum Fügen einer Vielzahl von Geweben, beispielsweise für parenchymatöse Gewebe (u. a. Niere, Leber Lunge, Milz, Hirn, Fettgewebe), kollagenhaltige Gewebe (u. a. Knorpel, Blutgefässe, Harnleiter, Sehnen, Lymphgefäße), Körpermembranen (u. a. Haut, Zwerchfell, Blase, Magenwand, Gebärmutterwand, Hirnhaut, Rückenmark-Haut, Hornhaut des Auges, Netzhaut), myelinhaltige Gewebe (u. a. Nervenfasern), Muskelgewebe, Knochen. Beim Fügen zweier Gefäße kann die Heizstrahlung beispielsweise mittels eines Lichtwellenleiters in einem der beiden Gefäße bis an die Fügestelle herangeführt und dort beispielsweise mittels eines reflektierenden Kegels oder eines Diffusors in einen ringförmigen Raumbereich verteilt werden.

Jenseits der oben beschriebenen human- oder veterinärmedizinischen Anwendungen ist die vorliegende Erfindung auch für nicht-medizinische Anwendungen aus verschiedenen Gebieten der Technik geeignet. Beispielsweise können mit der vorliegenden Erfindung beim Löten von Kunststoffen gleichmäßigere Temperaturen erzielt und eine lokale oder großräumige Überhitzung der zu fügenden Teile verhindert werden.

### Bezugszeichen

- 10: Hohlraum
- 11: erstes Teil
- 12: zweites Teil
- 13: Lot
- 16: Oberfläche
- 17: Oberfläche
- 19: Öffnung im ersten Teil 11
- 20: Endoskop
- 21: Lichtquelle im Endoskop 20
- 22: Objektiv im Endoskop 20
- 23: Bildsensor im Endoskop 20
- 26: Beleuchtungsleitung im Endoskop 20
- 27: Bildleitung im Endoskop 20
- 30: Steuerung
- 36: Beleuchtungsausgang der Steuerung 30
- 37: Bildsignaleingang der Steuerung 30
- 39: Benutzerschnittstelle
- 41: Heizstrahlung
- 42: gestreute, emittierte oder reflektierte Strahlung
- 50: Kapsel
- 51: Hülle der Kapsel 50
- 52: Thermochrom der Kapsel 50
- 60: Kontraktionseinrichtung
- 101: erster Schritt
- 102: zweiter Schritt
- 103: dritter Schritt
- 104: vierter Schritt
- 105: fünfter Schritt
- 106: sechster Schritt
- 107: siebter Schritt
- 108: achter Schritt
- 109: neunter Schritt
- 110: zehnter Schritt
- 111: erster Schritt
- 112: zweiter Schritt
- 113: dritter Schritt
- 114: vierter Schritt
- 115: fünfter Schritt

## Patentansprüche

1. Nichtmetallisches Lot (13) zum stoffschlüssigen Fügen zweier Oberflächen (16, 17) eines menschlichen oder tierischen Körpers durch Benetzen beider Oberflächen (16, 17) durch das geschmolzene Lot (13) und Verbinden der Oberflächen (16, 17) durch das erstarrte Lot (13), mit:
einer reversibel oder irreversibel temperaturabhängigen optischen Eigenschaft für elektromagnetische Strahlung mit einem vorbestimmten Spektrum im infraroten Spektralbereich, wobei
das Lot (13) ein für elektromagnetische Strahlung mit dem vorbestimmten Spektrum transparentes Polymer und zumindest entweder thermochrome Pigmente oder einen thermochromen Farbstoff aufweist.

2. Lot (13) nach Anspruch 1, wobei das Lot (13) eine temperaturabhängige Absorptionskonstante für elektromagnetische Strahlung mit einem vorbestimmten Spektrum aufweist.

3. Lot (13) nach einem der Ansprüche 1 und 2, ferner mit:
Kapseln (50) mit einer Hülle (51), die für elektromagnetische Strahlung mit dem vorbestimmten Spektrum transparent ist und ein thermochromes Pigment (52) oder einen thermochromen Farbstoff umschließt.

4. Lot (13) nach einem der Ansprüche 1 bis 3, wobei das Lot (13)
eine temperaturabhängige Absorptionskonstante für elektromagnetische Strahlung mit einem vorbestimmten Spektrum im infraroten Spektralbereich aufweist, die sich bei einer ersten vorbestimmten Temperatur ändert,
ein temperaturabhängiges Absorptions-, Streu- oder Fluoreszenzverhalten im sichtbaren Bereich aufweist, das sich bei der ersten vorbestimmten Temperatur oder bei einer zweiten vorbestimmten Temperatur ändert.

5. Steuerung (30) zum Steuern eines Schmelzens eines nichtmetallischen Lots (13) mit einer temperaturabhängigen optischen Eigenschaft für elektromagnetische Strahlung mit einem vorbestimmten Spektrum im infraroten Spektralbereich mit:
einem Eingang (37) zum Erfassen eines Signals eines Sensors (23) für eine Intensität oder Leistung der von dem Lot (13) transmittierten oder reflektierten oder gestreuten elektromagnetischen Strahlung (42) und
einem Beleuchtungsausgang (36)
wobei die Steuerung dazu ausgebildet ist, um die elektromagnetische Strahlung zum Schmelzen des nichtmetallischen Lots, bei Überschreitung oder Untarschreitung einer vorbestimmten Schwelle einer absoluten oder relativen Änderung der durch den Sensor erfassten Intensität oder Leistung zu beenden oder reduzieren

6. Vorrichtung zum stoffschlüssigen Fügen zweier Oberflächen (16, 17) in oder an einem menschlichen oder tierischen Körper, mit:
einer Steuerung (30) nach Anspruch 5;
einer Lichtquelle (21) zum Bestrahlen des Lots (13) mit elektromagnetischer Strahlung mit dem vorbestimmten Spektrum;
einem Sensor (23) zum Erfassen einer Intensität oder Leistung von elektromagnetischer Strahlung, die von dem Lot (13) transmittiert oder reflektiert oder gestreut ist.

## Claims

1. Non-metallic solder (13) for cohesively joining two surfaces (16, 17) of a human or animal body by wetting both surfaces (16, 17) by means of the molten solder (13) and connecting the surfaces (16, 17) by the solidified solder (13), comprising:
a reversibly or irreversibly temperature-dependent optical property for electromagnetic radiation with a predetermined spectrum in the infrared spectral range, wherein
the solder (13) has a polymer transparent to electromagnetic radiation with the predetermined spectrum and at least has either thermochromic pigments or a thermochromic dye.

2. Solder (13) according to Claim 1, wherein the solder (13) has a temperature-dependent attenuation coefficient for electromagnetic radiation with a predetermined spectrum.

3. Solder (13) according to either of Claims 1 or 2, furthermore comprising:
capsules (50) with a sheath (51) which is transparent to electromagnetic radiation with the predetermined spectrum and surrounds a thermochromic pigment (52) or a thermochromic dye.

4. Solder (13) according to one of Claims 1 to 3, wherein the solder (13)
has a temperature-dependent attenuation coefficient for electromagnetic radiation with a predetermined spectrum in the infrared spectral range, which changes at a first predetermined temperature,
a temperature-dependent attenuation behaviour, scattering behaviour or fluorescence behaviour in the visible range, which changes at the first predetermined temperature or at a second predetermined temperature.

5. Control unit (30) for controlling a melting of a non-metallic solder (13) with a temperature-dependent optical property for electromagnetic radiation with a predetermined spectrum in the infrared spectral range, comprising:
an input (37) for detecting a signal from a sensor (23) for an intensity or power of the electromagnetic radiation (42) transmitted or reflected or scattered by the solder (13) and
an illumination output (36),
wherein the control is configured to terminate or reduce the electromagnetic radiation for melting the non-metallic solder if a predetermined threshold of an absolute or relative change in the intensity or power detected by the sensor is overshot or undershot.

6. Device for cohesively joining two surfaces (16, 17) in or on a human or animal body, comprising:
a control unit (30) according to Claim 5;
a light source (21) for irradiating the solder (13) with electromagnetic radiation with the predetermined spectrum;
a sensor (23) for detecting an intensity or power of electromagnetic radiation which is transmitted or reflected or scattered by the solder (13).

## Revendications

1. Soudure non métallique (13) destinée à rejoindre en correspondance de matière deux surfaces (16, 17) d'un corps humain ou animal par mouillage des deux surfaces (16, 17) par la soudure (13) fondue et par liaison des surfaces (16, 17) par la soudure (13) solidifiée,
la soudure présentant vis-à-vis d'un rayonnement électromagnétique d'un spectre prédéterminé dans la plage spectrale de l'infrarouge une propriété optique qui dépend de manière réversible ou irréversible de la température,
la soudure (13) présentant un polymère transparent au rayonnement électromagnétique présentant le spectre prédéterminé et au moins des pigments thermochromes ou un colorant thermochrome.

2. Soudure (13) selon la revendication 1, la soudure (13) présentant une constante d'absorption du rayonnement électromagnétique du spectre prédéterminé qui dépend de la température.

3. Soudure (13) selon l'une des revendications 1 et 2, et présentant en outre des capsules (50) dotées d'une enveloppe (51) transparente vis-à-vis du rayonnement électromagnétique de spectre prédéterminé et qui englobent un pigment thermochrome (52) ou un colorant thermochrome.

4. Soudure (13) selon l'une des revendications 1 à 3, dans laquelle la soudure (13) présente une constante d'absorption du rayonnement électromagnétique dans le spectre prédéterminé dans la plage spectrale de l'infrarouge qui dépend de la température et qui se modifie à une première température prédéterminée et un comportement d'absorption, de diffusion ou de fluorescence dans la plage visible qui dépend de la température et qui se modifie à la première température prédéterminée ou à une autre température prédéterminée.

5. Commande (30) destinée à commander la fusion d'une soudure non métallique (13) qui présente vis-à-vis d'un rayonnement électromagnétique qui présente un spectre prédéterminé dans la plage spectrale de l'infrarouge une propriété optique dépendant de la température, la commande présentant une entrée (37) qui saisit un signal d'un capteur (23) d'intensité ou de puissance du rayonnement électromagnétique (42) transmis, réfléchi ou diffusé par la soudure (13) et une sortie d'éclairage (36), la commande étant configurée pour interrompre ou réduire le rayonnement électromagnétique servant à faire fondre la soudure non métallique lorsqu'un seuil prédéterminé d'une modification absolue ou relative de l'intensité ou de la puissance saisie par le capteur est dépassé ou n'est pas atteint.

6. Dispositif de jonction en correspondance de matière de deux surfaces (16, 17) dans ou sur un corps humain ou animal, le dispositif présentant :
une commande (30) selon la revendication 5,
une source de lumière (21) qui irradie la soudure (13) par un rayonnement électromagnétique de spectre prédéterminé et
un capteur (23) qui saisit l'intensité ou la puissance du rayonnement électromagnétique qui est transmis, réfléchi ou diffusé par la soudure (13).
